Europäisches Patentamt

European Patent Office

Office européen des brevets

(19)

(11) Publication number: **0 228 228**
**A2**

# EUROPEAN PATENT APPLICATION

(21) Application number: **86309766.3**

(22) Date of filing: **15.12.86**

(51) Int. Cl.⁴: **C 12 N 1/20,** C 12 P 21/00,
A 01 N 63/00
//
(C12N1/20, C12R1:07),
(C12P21/00, C12R1:07)

(30) Priority: **16.12.85 US 809556**

(43) Date of publication of application: **08.07.87**
**Bulletin 87/28**

(84) Designated Contracting States: **BE CH DE FR GB IT LI
NL SE**

(71) Applicant: **Mycogen Corporation, 5451 Oberlin Drive,
San Diego, CA 92121 (US)**

(72) Inventor: **Hermstadt, Corinna, 8448 New Salem Street,
No. 6, San Diego California 92126 (US)**
Inventor: **Gaertner, Frank H., 4465 Ocean Blvd., No. 36,
San Diego California 92109 (US)**

(74) Representative: **Perry, Robert Edward et al, GILL
JENNINGS & EVERY 53-64 Chancery Lane, London
WC2A 1HN (GB)**

(54) **Mutants of bacillus thuringiensis.**

(57) Stable spo⁻ cry⁺ mutants of *B. thuringiensis* have been prepared, efficiently and predictably, by contacting or treating *B. thuringiensis* microorganisms with ethidium bromide. The mutants have advantages over the parent spore-forming organisms, e.g. greater efficiency at producing toxic crystal which is useful as a pesticide. A stable spo⁻ cry⁺ mutant of *B. thuringiensis* M-7 produces a crystal toxic to beetles of the order *Coleoptera*.

# MUTANTS OF BACILLUS THURINGIENSIS

Isolation of asporogenous (spo⁻) mutants in Bacillus species is made difficult by the lack of selective techniques for the spo⁻ condition. Also a high frequency of a type of leaky mutation, termed oligosporogenous, complicates the isolation procedure. Oligosporogenous strains appear at first to be asporogenous, but upon closer inspection they can be seen to produce spores at a low frequency. Rogolsky and Slepecky (Rogolsky, M. and Slepecky, R.A. [1968] Canadian J. of Microb. 14:61-64) noted that a DNA-intercalating dye, acriflavin, induced what they believed to be a high proportion of asporogenic mutants in growing cultures of Bacillus subtilis (up to 44%). Consequently they proposed that some genetic component involved in Bacillus sporulation is carried on an episome that can be eliminated by the curing action of acriflavin. However, Schaeffer (Schaeffer, P. [1969] Bacteriol. Rev. 33:48-71] was unable to repeat their work, and genetic mapping localized all of the B. subtilis spo⁻ genes on the Bacillus chromosome. Due to these conflicting results, work on the effect of acriflavin was

-2-

not pursued.

The above art is solely concerned with B. subtilis organisms. Though such work could be extrapolated to other Bacillus species where asporogenous mutants are desired, it does not address the problem of making asporogenous crystal-producing ($spo^- cry^+$) mutants of strains of B. thuringiensis. The introduction of toxic crystal production complicates the entire situation because there is an uncertainty surrounding the linkage of genes for spore production with the one(s) associated with crystal production. There is no known prior art which discloses or suggests a predictable efficient process for preparing stable $spo^- cry^+$ mutants of B. thuringiensis. The need for such mutants is clear, as shown infra.

## Brief Summary of the Invention

The subject invention concerns a predictable efficient process for preparing stable $spo^- cry^+$ mutants of Bacillus thuringiensis. More specifically, upon treating a spore-forming, toxic crystal-producing Bacillus thuringiensis with ethidium bromide there is obtained a stable $spo^- cry^+$ B. thuringiensis mutant. The process is exemplified by treating B. thuringiensis M-7 (NRRL B-15939), a sporogenous toxic crystal-producer, with ethidium bromide to give a B. thuringiensis M-7 mutant which is $spo^-$ and $cry^+$. This mutant is useful to produce a toxic crystal which is active against beetles of the order Coleoptera.

The subject process also can be used to make stable $spo^- cry^+$ mutants of other B. thuringiensis strains. A representative list of these B. thuringiensis strains is disclosed infra.

-3-

Further, the subject invention concerns the use of the stable spo$^-$ cry$^+$ mutants as pesticides.

The stable asporogenous crystal-producer strains of B. thuringiensis have several advantages:

(1) Contamination of fermentation facilities with bacterial spores, which is a serious problem in facilities where other fermentations are conducted, is non-existent.

(2) Without a spore to produce, B. thuringiensis is more efficient at producing pesticidal-crystal protein.

(3) With the fixed-cell procedure, where dead cells are the goal as a pesticide (especially in the case of recombinant bacilli) the spores will not be there to resist the fixation process and consequently the cells will be dead.

(4) Where intact B. thuringiensis cells are wanted as a pesticide (as in the fixed-cell procedure), there is less tendency for the cells to lyse during the fermentation process.

## Detailed Description of the Invention

Upon treating a living population of sporogenous toxic crystal-producing B. thuringiensis with ethidium bromide there are obtained stable spo$^-$ cry$^+$ B. thuringiensis mutants. For example, upon culturing B. thuringiensis M-7 in the presence of ethidium bromide, there is obtained a stable spo$^-$ cry$^+$ mutant.

The B. thuringiensis cells, advantageously, are grown in the presence of ethidium bromide. Alternatively, living B. thuringiensis cells can be treated

-4-

by adding ethidium bromide to a suspension of cells for a time sufficient to produce the desired mutant. When B. thuringiensis cells are grown in the presence of ethidium bromide, the cells are generally grown for about 3 to about 7 days, at about 28 to about 32°C.

Detection of spo$^-$ cry$^+$ mutants is done by procedures well known to those skilled in the art. For example, colonies can be grown on L-broth (Bacto tryptone, 10 g/l; Bacto yeast, 5 g/l; NaCl, 5 g/l; ampicillin, 50 mg/l) and on SCG plates (0.1% vitamin free casamino acids, 0.5% glucose, 0.2% ammonium sulfate, 1.4% dipotassium phosphate, 0.5% monopotassium phosphate, 0.1% sodium citrate, 0.02% magnesium sulfate.) The medium stimulates sporulation in bacilli.

Other B. thuringiensis strains which can be treated in accord with the procedures disclosed herein are as follows:

Bacillus thuringiensis var. kurstaki HD1--
NRRL B-3792; disclosed in U.S. Patent 4,448,885

Bacillus thuringiensis var. israelensis--ATCC 35646

Bacillus thuringiensis var. tenebrionis--DSM 2803

The following B. thuringiensis cultures are available from the United States Department of Agriculture (USDA) at Brownsville, Texas. Requests should be made to Joe Garcia, USDA, ARS, Cotton Insects Research Unit, P.O. Box 1033, Brownsville, Texas 78520 USA.

B. thuringiensis var. thuringiensis HD2

B. thuringiensis var. finitimus HD3

B. thuringiensis var. alesti HD4

B. thuringiensis var. kurstaki HD73

B. _thuringiensis_ var. _sotto_ HD770
B. _thuringiensis_ var. _dendrolimus_ HD7
B. _thuringiensis_ var. _kenyae_ HD5
B. _thuringiensis_ var. _galleriae_ HD29
B. _thuringiensis_ var. _canadensis_ HD224
B. _thuringiensis_ var. _entomocidus_ HD9
B. _thuringiensis_ var. _subtoxicus_ HD109
B. _thuringiensis_ var. _aizawai_ HD11
B. _thuringiensis_ var. _morrisoni_ HD12
B. _thuringiensis_ var. _ostriniae_ HD501
B. _thuringiensis_ var. _tolworthi_ HD537
B. _thuringiensis_ var. _darmstadiensis_ HD146
B. _thuringiensis_ var. _toumanoffi_ HD201
B. _thuringiensis_ var. _kyushuensis_ HD541
B. _thuringiensis_ var. _thompsoni_ HD542
B. _thuringiensis_ var. _pakistani_ HD395
B. _thuringiensis_ var. _israelensis_ HD567
B. _thuringiensis_ var. _indiana_ HD521
B. _thuringiensis_ var. _dakota_
B. _thuringiensis_ var. _tohokuensis_ HD866
B. _thuringiensis_ var. _kumanotoensis_ HD867
B. _thuringiensis_ var. _tochigiensis_ HD868
B. _thuringiensis_ var. _colmeri_ HD847
B. _thuringiensis_ var. _wuhanensis_ HD525

B. _thuringiensis_ M-7, exemplified herein, is a B. _thuringiensis_ isolate which, surprisingly, has activity against beetles of the order Coleoptera but not against _Trichoplusia_ _ni_, _Spodoptera_ _exigua_ or _Aedes_ _aegypti_. Included in the Coleoptera are various _Diabrotica_ species (family Chrysomelidae) that are responsible for large agricultural losses, for example, D. undecimpunctata (western spotted cucumber beetle), D. longicornis (northern corn rootworm), D. virgitera (western corn rootworm), and D. undecimpunctata howardi (southern corn rootworm).

B. thuringiensis M-7 is unusual in having a unique parasporal body (crystal) which under phase contrast microscopy is dark in appearance with a flat, square configuration.

The parent B. thuringiensis can be cultured using standard art media and fermentation techniques. Upon completion of the fermentation cycle, the bacteria can be harvested by means well known in the art.

The stable spo$^-$ cry$^+$ mutants producible by the process of the subject invention are useful as pesticides. For example, the mutant B. thuringiensis can be treated with reagents that prolong the activity of the toxin produced in the cell when the cell is applied to the environment of target pest(s). The resulting product retains the toxicity of the toxin.

The pesticide can be any toxin produced by a microbe. For example, it can be a polypeptide which has toxic activity toward a eukaryotic multicellular pest, such as insects, e.g., coleoptera, lepidoptera, diptera, hemiptera, dermaptera, and orthoptera; or arachnids; gastropods; or worms, such as nematodes and platyhelminths. Various susceptible insects include beetles, moths, flies, grasshoppers, lice, and earwigs.

0228228

-7-

The pesticide made in the host cell can be a polypeptide produced in active form or a precursor or pro-
form requiring further processing for toxin activity,
e.g., the crystal toxin of B. thuringiensis var. kurstaki, which requires processing by the pest.

The method of treating the organism can fulfill
a number of functions. First, it may enhance structural
integrity. Second, it may provide for enhanced proteolytic stability of the toxin, by modifying the toxin so
as to reduce its susceptibility to proteolytic degradation and/or by reducing the proteolytic activity of
proteases naturally present in the cell. The cells
are preferably modified at an intact stage and when
there has been a substantial build-up of the toxin
protein. These modifications can be achieved in a
variety of ways, such as by using chemical reagents
having a broad spectrum of chemical reactivity. The
intact cells can be combined with a liquid reagent
medium containing the chemical reagents, with or without
agitation at temperatures in the range of about -10 to
60°C. The reaction time may be determined empirically
and will vary widely with the reagents and reaction
conditions. Cell concentrations will vary from about
$10E^2$ to $10E^{10}$ per ml.

Of particular interest as chemical reagents are
halogenating agents, particularly halogens of atomic
no. 17-80. More particularly, iodine can be used under
mild conditions and for sufficient time to achieve the
desired results. Other suitable techniques include
treatment with aldehydes, such as formaldehyde and
glutaraldehyde; anti-infectives, such as zephiran
chloride and cetylpyridinium chloride; alcohols, such
as 2-propanol and ethanol; various histologic fixatives,

-8-

such as Bouin's fixative and Helly's fixative (See: Humason, Gretchen L., Animal Tissue Techniques, W.H. Freeman and Company, 1967); or a combination of physical (heat) and chemical agents that prolong the activity of the toxin produced in the cell when the cell is applied to the environment of the target pest(s).

For halogenation with iodine, temperatures will generally range from about 0 to 50°C, but the reaction can be conveniently carried out at room temperature. Conveniently, the iodination may be performed using triiodide or iodine at 0.5 to 5% in an acidic aqueous medium, particularly an aqueous carboxylic acid solution that may vary from about 0.5-5M. Conveniently, acetic acid may be used, although other carboxylic acids, generally of from about 1 to 4 carbon atoms, may also be employed. The time for the reaction will generally range from less than a minute to about 24 hrs, usually from about 1 to 6 hrs. Any residual iodine may be removed by reaction with a reducing agent, such as dithionite, sodium thiosulfate, or other reducing agent compatible with ultimate usage in the field. In addition, the modified cells may be subjected to further treatment, such as washing to remove all of the reaction medium, isolation in dry form, and formulation with typical stickers, spreaders, and adjuvants generally utilized in agricultural applications, as is well known to those skilled in the art.

Of particular interest are reagents capable of crosslinking the cell wall. A number of reagents are known in the art for this purpose. The treatment should result in enhanced stability of the pesticide. That is, there should be enhanced persistence or residual activity of the pesticide under field conditions. Thus, under conditions where the pesticidal activity of untreated cells diminishes, the activity of treated cells remains for periods of from 1 to 3 times longer.

The cells can be formulated for use in the environment in a variety of ways. They can be employed as wettable powders, granules, or dusts, by mixing with various inert materials, such as inorganic minerals (phyllosilicates, carbonates, sulfates, or phosphates) or botanical materials (powdered corncobs, rice hulls, or walnut shells). The formulations can include spreader/sticker adjuvants, stabilizing agents, other pesticidal additives, or surfactants. Liquid formulations can be aqueous-based or non-aqueous and employed as foams, gels, suspensions, emulsifiable concentrates, and the like. The ingredients can include rheological agents, surfactants, emulsifiers, dispersants, polymers, and the like.

The pesticidal concentration will vary depending upon the nature of the particular formulation, e.g., whether it is a concentrate or to be used undiluted. The pesticide will generally be present at a concentration of at least about 1% by weight, but can be up to 100% by weight. The dry formulations will have from about 1 to 95% by weight of the pesticide, while the liquid formulations will generally be from about 1 to 60% by weight of the solids in the liquid phase. The formulations will generally have from about 1E2 to 1E8 cells/mg.

The formulations can be applied to the environment of the pest(s), e.g., plants, soil or water, by spraying, dusting, sprinkling or the like. These formulations can be administered in an amount (liquid or dry) of at least 0.05, e.g. up to 1, kg/hectare, as required.

The following Examples illustrate the invention.

Example 1

A culture of B. thuringiensis M-7 cells in L-broth containing 2-5 µg ethidium bromide/ml was grown in the dark for 4 days at 30°C. The culture was diluted and plated on L-broth agar plates in order to isolate single colonies. Single colonies were picked and analysed by phase contrast light microscopy for deficiency of either spore or crystal or both. All colonies were checked on L-broth and SCG plates. $Spo^-$ $cry^+$ mutants were isolated with an efficiency of 9-12%.

A subculture of a stable $spo^-$ $cry^+$ mutant of B. thuringiensis M-7 was deposited in the permanent collection of the Northern Regional Research Laboratory, U.S. Department of Agirculture, Peoria, Illinois, USA, on 15th November 1985. The culture was assigned the accession number NRRL B-18029.

Formulated products of stable $spo^-$ $cry^+$ mutants of B. thuringiensis M-7 can be sprayed or applied on to foliage to control phytophagous beetles. Formulated bait granules containing an attractant and spores and crystals of mutant B. thuringiensis M-7 can be applied to the soil for control of soil-inhibiting Coleoptera. Formulated mutant B. thuringiensis M-7 can also be applied as a seed-coating or root treatment or total plant treatment.

Against Diabrotica larvae which feed on the roots of corn, a bait granule containing cucurbitacin (a phagostimulant for diabroticite beetles derived from cucurbits) or other insect attractant and spores and crystals of mutant B. thuringiensis M-7 can be formulated. These granules can be planted in the row at planting. Further, a formulated, e.g. a wettable powder, can be sprayed directly on to foliage, to control susceptible adult beetles.

Tests have shown that B. thuringiensis M-7, NRRL B-15939, is active against the yellow mealworm, Tenebrio molitor. This beetle is a species of the family Tenebrionidae which is in the order Coleoptera. Thus, mutant B. thuringiensis M-7 can be used to control this beetle.

Example 2

By culturing a strain of Bacillus thuringiensis var. aizawai (A.W. West (1984) Soil Biol. Biochem. 16:357-360) as in Example 1, stable spo$^-$ cry$^+$ mutants are obtained.

B. thuringiensis M-7 is described and claimed in EP-A-0192319.

-12-

CLAIMS

1. A stable spo$^-$ cry$^+$ mutant of <u>Bacillus thuringiensis</u>.

2. A mutant according to claim 1, wherein the <u>B</u>. <u>thuringiensis</u> is selected from var. <u>kurstaki</u>, var. <u>israelensis</u>, M-7, var. <u>finitimus</u>, var. <u>alesti</u>, var. <u>sotto</u>, var. <u>dendrolimus</u>, var. <u>kenyae</u>, var. <u>galleriae</u>, var. <u>canadensis</u>, var. <u>entomocidus</u>, var. <u>subtoxicus</u>, var. <u>aizawai</u>, var. <u>morrisoni</u>, var. <u>ostriniae</u>, var. <u>tolworthi</u>, var. <u>darmstadiensis</u>, var. <u>toumanoffi</u>. var. <u>kyushuensis</u>, var. <u>thompsoni</u>, var. <u>pakistani</u>, var. <u>indiana</u>, var. <u>dakota</u>, var. <u>tohokuensis</u>, var. <u>kumanotoensis</u>, var. <u>tochigiensis</u>, var. <u>colmeri</u>, var. <u>wuhanensis</u>, var. <u>tenebrionis</u> and var. <u>thuringiensis</u>.

3. A mutant according to claim 1, of <u>B</u>. <u>thuringiensis</u> M-7.

4. Crystals obtainable from a mutant according to claim 3.

5. A process for preparing a mutant according to any of claims 1 to 3, which comprises treating <u>B</u>. <u>thuringiensis</u> with ethidium bromide.

6. A composition which comprises crystals according to claim 4 and an attractant.

7. A composition according to claim 6, wherein the attractant is cucurbitacin.

8. A composition which comprises a mutant according to claims 1 to 3, which is in the form of a bait granule or seed dressing.

9. A method for controlling susceptible insect pests, which comprises applying to the pests or to their locus a mutant according to any of claims 1 to 3, crystals according to claim 4, or a composition according to any of claims 6 to 8.

10. A method according to claim 9, wherein the insect pests belong to the order <u>Coleoptera</u>.

11. A method according to claim 8 or claim 9, which comprises placing a bait granule including crystals or the mutant of claim 3 or claim 4 on or in the soil at or about the time when planting seeds of a plant upon which the insect pests are known to feed.

12. A method according to claim 11, which comprises placing a bait granule including crystals according to claim 4 on or in the soil at the same time as planting corn seeds.

European Patent Office

**0228228**

Application number:

26 30276 3

# DECLARATION PURSUANT TO RULE 28, PARAGRAPH 4, OF THE EUROPEAN PATENT CONVENTION

The applicant has informed the European Patent Office that, until the publication of the mention of the grant of the European patent or until the date on which the application has been refused or withdrawn or is deemed to be withdrawn, the availability of the micro-organism(s) identified below, referred to in paragraph 3 of Rule 28 of the European Patent Convention, shall be effected only by the issue of a sample to an expert.

## IDENTIFICATION OF THE MICRO-ORGANISMS

**Accession numbers of the deposits:**

NRRL - B - 18029